# EUROPEAN PATENT APPLICATION

(11) **EP 1 508 331 A1**
(43) Date of publication of application: **23.02.2005**
(21) Application number: 03755278.3
(22) Date of filing: 21.05.2003
(51) Int. Cl.: A61K 9/107, A61K 9/19, A61K 31/337, A61K 31/407, A61K 31/4745, A61K 31/519, A61K 31/557, A61K 31/704, A61K 31/7048, A61K 33/24, A61K 47/34, A61K 47/26, A61K 47/36, A61P 35/00

(54) **METHOD FOR PREPARING POLYMER MICELLE PHARMACEUTICAL PREPARATION CONTAINING DRUG FOR INJECTION**

(30) Priority: 24.05.2002 JP 2002150890
(71) Applicant: NanoCarrier Co., Ltd., Kashiwa-shi, Chiba-ken 277-0882 (JP)
(72) Inventor: NAGASAKI, Shoko, Moriya-shi, Ibaraki 302-0128 (JP); TSUCHIYA, Chieko, Nerima-ku, Tokyo 177-0043 (JP); SAGAWA, Katsuhiko Naka-Namerikawa City-Heights 322, Toyama 936-0056 (JP)
(74) Representative: Albrecht, Thomas, Dr.
(86) International application number: PCT/JP2003/006334
(87) International publication number: WO 2003/099260

(57) **Abstract**

A production method of a preparation containing drug-encapsulating polymer micelles is provided, which comprises dissolving a hydrophilic-hydrophobic block copolymer and a sparingly water-soluble drug in a volatile organic solvent, then removing the solvent, and stirring the residue with water at a temperature not higher than 30°C to dissolve the drug-encapsulating polymer micelles into the water.

## Description

### Technical Field

This invention relates to a method for preparing a polymer micelle pharmaceutical preparation containing sparingly water-soluble drug, and to lyophilized preparations obtainable by said preparation method.

### Background Art

As the typical of preparation methods of polymer micelles which encapsulate sparingly water-soluble drug, using block copolymers having hydrophilic segments and hydrophobic segments, the following methods a) , c) which are described in JP 2777530 and method d) which is described in JP 2001-226294A are known.

### a) Drug-encapsulating method by agitation

A sparingly water-soluble drug, as dissolved in water-miscible organic solvent where necessary, is mixed with an aqueous dispersion-solution of said block copolymer and stirred. In occasions, heating may be applied at the time of mixing and stirring, to promote encapsulation of the drug in the polymer micelles.

### b) Solvent-volatilization method

A water-immiscible organic solvent solution of a sparingly water-soluble drug and an aqueous dispersion-solution of said block copolymer are mixed and the organic solvent is volatilized under stirring.

### c) Dialysis method

A sparingly water-soluble drug and said block copolymer are dissolved in a water-miscible organic solvent, and the resulting solution is dialyzed against buffer and/or water, using a permeable membrane.

### d) Others

A sparingly water-soluble drug and said block copolymer are dissolved in a water-immiscible organic solvent, the resulting solution is mixed with water, stirred to form an oil-in-water (O/W) type emulsion, and the solvent is volatilized.

Furthermore, a method which is normally referred to as solid dispersion method or solvent evaporation method is also known for preparation of drug-encapsulating polymer micelle solutions, which comprises dissolving said drug and block copolymer in an organic solvent, distilling the solvent off after homogeneously mixing the two, and dissolving the solid homogeneous mixture in water at 60°C or 40°C (cf. e.g., Park et al., Biomaterials and Drug Delivery toward New Mellenium, 2000, 321-332; Lavasanifar et al., Journal of Controlled Release, 77 (2001), 155-160). In these methods, liquids containing drug-encapsulating polymer micelles of a size passing through filter of 0.22 µm in pore size, which is commonly used for sterilizing filtration, are obtained. For example, according to the latter of the above-cited publications, drug (amphotericin B)-encapsulating polymer micelles can be obtained at an yield of 73%, and it is also disclosed that said micelle solution is filtered through a filter (0.22 µm), lyophilized, the resulting lyophilization product is re-dissolved (reconstituted) with water and filtered again (0.22 µm). A prospect for using such a re-dissolved or reconstituted liquid as an injection is also suggested. In these prior art methods, furthermore, dichloromethane is generally used as the organic solvent. Whereas, considering that the formulated medical preparations are directly applied to human, use of dichloromethane or the like which are suspected to be toxic to living organism is better avoided.

### Disclosure of the Invention

According to the above method by Lavasanifar et al., considerably high drug-encapsulating efficiency into polymer micelles is said to have been achieved.

However, it is still necessary to provide, in the occasions of encapsulating in polymer micelles drugs that are generally very expensive, drug-encapsulating polymer micelles having a desired polymer micelle size and, in addition, exhibiting high drug-encapsulating efficiency. Besides, those heretofore known methods generally use dichloromethane which is said to be toxic to living organism. Accordingly, therefore, the object of the present invention is to provide an improved preparation method of drug-encapsulating polymer micelles of a controlled particle size; or a method for making preparations containing such polymer micelles; or a preparation method in which a solvent free of apprehension of being toxic, or free of toxicity, to living organism, can be used.

We have engaged in multilateral investigations in preparation methods of drug-encapsulating polymer micelles with the view to accomplish the above object, to discover the following in consequence: when the dispersing temperature of a homogeneous solid mixture of a drug with a specific block copolymer uniformly in water, in the above-described solid dispersion method or solvent evaporation method, is set not higher than 30°C, yield of drug-encapsulating polymer micelles of desired particle size, in particular, such a particle size as can be used for injection as it is, could be increased definitely and significantly. That is, we confirmed that the critical point in the operation temperature for raising the efficiency of said methods lies in the vicinity of around 30°C.

Accordingly, the present invention provides a method for making a preparation containing drug-encapsulating polymer micelles with a controlled size, which comprises forming a solution by dispersing and dissolving a block copolymer with hydrophilic and hydrophobic segments, and a sparingly water-soluble drug in a volatile organic solvent, removing the organic solvent, joining the resultant residue to water, and stirring the same at a temperature not higher than 30°C for a time sufficient to uniformly disperse said residue in the water.

According to said method, drug-encapsulating polymer micelles which can pass through a filter of 0.22 µm in pore size, i.e., the filter normally used for sterilizing filtration for formulating injectable preparations, can be obtained with the drug-encapsulting effectivity exceeding 73%. According to the present invention, therefore, aqueous liquids of the drug-encapsulating polymer micelles which are provided by the method of the invention and passed through a 0.22 µm-filter can be used as they are as, for example, injections. On the other hand, the drug not utilized for the injections, if present at all, is negligible, hardly requiring recovery, because the polymer micelles unable to pass said 0.22 µm-filter are only of little quantity.

It is also an embodiment of the present invention, to optionally add to the aqueous liquid containing the drug-encapsulating polymer micelles, before it is passed through a 0.22 µm-filter, a buffer and other adjuvant(s). When saccharides or polyethylene glycols of specific molecular weight (also referred to as MACROGOL in the pharmacopoeia) are used as such adjuvants, lyophilization of the filtrates resulting from the 0.22 µm-filtration gives corresponding lyophilized preparations which produce an additional effect of inhibiting aggregation among the polymer micelles in their re-dissolved or reconstituted aqueous liquids. Considering the fact that such aggregation is frequently inavoidable depending on the kind of the drug, the embodiment of adding such adjuvant(s) is very important.

According to the present invention, a lyophilized preparation which can be obtained through the above-described production method and which, when reconstituted into an aqueous liquid, scarcely causes aggregation of the polymer micelles therein is also provided.

Hereinafter the invention is explained in detail.

A block copolymer comprising hydrophilic segment and hydrophobic segment, which is useful for the invention, may be any that can form a polymer micelle having a hydrophobic core domain and a hydrophilic shell domain in an aqueous liquid or medium, in the concurrent presence of a sparingly water-soluble drug and that suits the purpose of the invention. Although not in limitative sense, as examples of hydrophilic segment in such a block copolymer, poly(ethylene glycol) [which is also referred to as poly(ethylene oxide)], poly(malic acid), poly(saccharide), poly(acrylic acid), poly(vinyl alcohol) and the like can be named. Whereas, as examples of hydrophobic segment, poly(β-alkylaspartate), poly(β-alkylaspartate-co-aspartic acid), poly(β-aralkylaspartate), poly(β-aralkylaspartate-co-aspartic acid), poly(β-alkylaspartamide), poly(β-alkylaspartamide-co-aspartic acid), poly(β-aralkylaspartamide), poly(β-aralkylaspartamide-co-aspartic acid), poly(γ-alkylglutamate), poly(γ-alkylglutamate-co-glutamic acid), poly(γ-aralkylglutamate), poly(γ-aralkylglutamate-co-glutamic acid), poly(γ-alkylglutamide), poly(γ-alkylglutamide-co-glutamic acid), poly(γ-aralkylglutamide), poly(γ-aralkylglutamide-co-glutamic acid), poly(lactide), poly(lactide-co-glycolide), poly(ε-caprolactone), poly(δ-valerolactone) and poly(γ-butyrolactone) can be named. Furthermore, alkyl and aralkyl in the above-named segments have the following significations, respectively. "Alkyl" includes C₁-C₂₂ straight or branched chain alkyl, examples of which being lower alkyl such as methyl, ethyl, n-propyl, i-propyl, n-butyl, t-butyl and n-hexyl; medium alkyl containing more carbons; and tetradecyl, hexadecyl, octadecyl, docosanyl and the like. These groups may optionally be substituted with one or more halogen (e.g., fluorine, chlorine, bromine), and said medium or higher alkyl may be substituted with single hydroxyl group. As aralkyl, phenyl- C₁-C₄ alkyl, e.g., benzyl, can be named, which may be substituted with 1-3 halogen atoms or lower alkyl groups on the benzene ring.

Such polymer segments can be obtained by the means known per se, e.g., through ester or amide-interchange between benzyl group in poly(β-benzylaspartate) or poly(γ-benzylglutamate) and the corresponding alcohol or amine. Where a hydrophobic polymer segment is indicated as a copolymer, it can be obtained by partially esterifying or partially hydrolyzing the corresponding alkyl- or aralkyl-ester or amide. The extent of partial esterification can generally be 20-80%. The aspartic acid, glutamic acid and lactide may show optical activity of either type or may be a mixture of optical isomers.

Such hydrophilic segment and hydrophobic segment are not subject to any limitation in size, so long as they are capable of forming polymer micelles in aqueous liquid (or aqueous medium) in the concurrent presence of sparingly water-soluble drug. Whereas, generally the recurring units of such hydrophilic segment are 30-1000, and those of the hydrophobic segment are 10-100. Aqueous liquid or aqueous medium said in this specification means water per se or a buffer solution or medium.

As particular examples of such block copolymers which are easy for preparation and which can be conveniently used in the invention, those which are expressed by the following formulae (I) and (II) may be named. 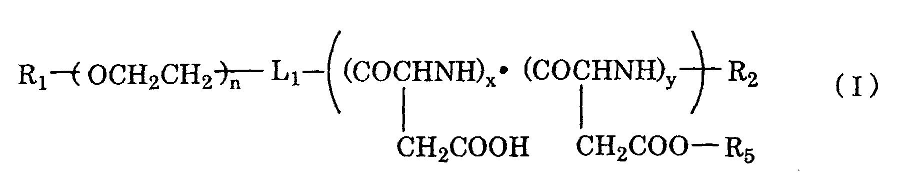 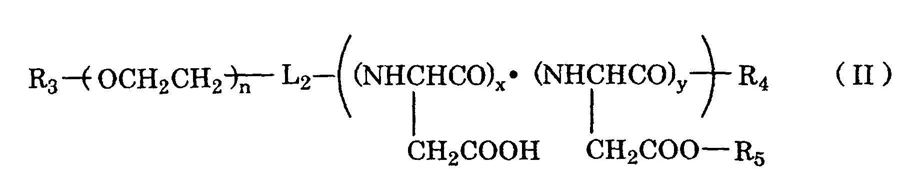 in the above formulae,
R₁ and R₃ each independently stands for hydrogen or an optionally protected functional group-substituted, or unsubstituted, lower alkyl,
R₂ stands for hydrogen, saturated or unsaturated C₁-C₂₉ aliphatic carbonyl or arylcarbonyl,
R₄ stands for hydroxyl, saturated or unsaturated C₁-C₃₀ aliphatic oxy or aryl-lower alkyloxy,
R₅ stands for benzyl or alkylbenzyl, or allyl,
L₁ and L₂ each independently stands for a linker,
n is an integer of 10-2500, and
x and y are same or different integers, their sum being 10-300,
x:y being within a range of 7:3-1:3, and x and y being present each at random.

Examples of optionally protected functional group include hydroxyl, acetal, ketal, aldehyde and sugar residues. The hydrophilic segments wherein R₁ and R₃ represent lower alkyl group(s) substituted with optionally protected functional group(s) can be those prepared by the methods described in, for example, WO96/33233, WO96/32434 and WO97/06202.

The linkers are variable depending mainly on individual production methods of the block copolymers and, therefore, are not critical. As specific examples, L₁ can be selected from a group consisting of-NH-, -O-, -CO-, -CH₂-, -O-Z-S-Z-, -OCO-Z-NH- and -O-Z-NH (wherein Z stands for C₁-C₄ alkylene, independently of each other); and L₂, selected from a group consisting of -OCO-Z-CO-, -NHCO-Z-CO- and -O-Z-NH (wherein Z stands for C₁-C₄ alkylene).

Sparingly water-soluble drug useful in the present invention signifies an organic compound or a conjugate thereof, whose solubility at normal temperature (25°C) is not higher than 0.5 mg per 1 ml of water, which, in accordance with the purpose of the invention, exhibits a certain pharmacological activity. As the typical examples of such drug, paclitaxel or derivatives thereof (e.g., docetaxel), camptothecin or derivatives thereof (e.g, irinotecan), cisplatin, daunorubicin, doxorubicin, methotrexate, mitomycin C, vincristine, amphotericin B, nystatin, prostaglandins and macrolide antibrotics can be named.

According to the method of the present invention, such a block copolymer and a drug are dispersed and dissolved in a volatile organic solvent. "Dispersed and dissolved" means not only the state of the block copolymer and the drug, which are the solute, being perfectly dissolved, but also such a state that they are solubilized and dispersed, for example, as polymer micelles. It should be noted that the term, "solution", as used in this specification in occasions encompasses the state of dispersion as above-described. The solvent useful for that purpose is not critical, so long as it can achieve the purpose with no apprehension about toxicity to living organism, examples of which include methyl alcohol, ethyl alcohol, isopropyl alcohol, acetone, acetonitrile, methyl acetate, ethyl acetate, tetrahydrofuran, diethyl ether, cyclohexane and the like; and mixed solvents of the foregoing; which are volatile (i.e., tend to gasify) at ambient temperature. Said dispersing and dissolving may be carried out under heating up to boiling point of the solvent, if permissible in respect of the characteristic properties of the particular drug used in the individual occasion, but normally the operation is conducted at not higher than ambient temperature but exceeding freezing point of the used solvent, by stirring of the solute to homogeneity.

After so forming a solution, the solvent is evaporated off, if necessary under reduced pressure. While complete removal of the solvent is not necessarily required in the present invention, the residue after the removal of the solvent should maintain pasty or solid condition. Whereas, when the subsequently obtained aqueous liquid containing the drug-encapsulating polymer micelles is to be used as an injection as it is, preferably the solvent is removed substantially completely.

Then the pasty or solid residue is combined with water (by adding water to the residue or vice versa), followed by stirring at a temperature not higher than 30°C, preferably not higher than 25°C, more preferably not higher than 10°C or 5°C. Where necessary, ultrasonic waves may be applied. This stirring is conducted for a time sufficient to achieve substantially completely uniform dispersion of the residue formed of the block copolymer and the drug. The time to achieve such uniform dispersion varies depending on the kinds of the polymer and the drug, and hence it is not critical, while generally preferred stirring time would be at least 5 hours but within 24 hours. The quantitative ratio of the residue and water may be 1:10 - 1:300.

Thus, drug-encapsulating polymer micelles are formed and are present in the aqueous solution. According to the method of the present invention wherein the solubilizing temperature is set at about 30°C, the drug-encapsulating polymer micelles have an average particle size of about 140 nm when measured with dynamic light-scattering photometer (Otsuka Electronics Co., DLS-7000DH model), and more than 73% of the polymer micelle particles pass through a filter of 0.22 mµ (220 nm) in pore size. Furthermore, when the temperature during the solubilization is set at about 25°C, the ratio of the polymer micelles passing through the 0.22 µm-filter generally exceeds about 80%; and when the temperature is set at about 10°C, that of the polymer micelles passing through the 0.22 µm-filter generally exceeds about 90%.

It is known that 0.22 µm-filters are normally used in the occasions of preparing injections (intravenous injection, intra-arterial injection, intramuscular injection, intra-abdominal injection and the like). Those aqueous solutions of drug-encapsulating polymer micelles provide, as above-described, sterilized drug-encapsulating polymer micelle aqueous solutions at very high yields, even when subjected to sterilizing filtration using such a 0.22 µm-filter. That is, according to the present invention, injectable preparations can be efficiently provided. As one of preferred embodiments of the present invention, such injections can be prepared by the method of the invention which comprises an additional step of adding adjuvants which can improve stability of the drug-encapsulating polymer micelles, such as various saccharides and various polyethylene glycols (tradename: MACROGOL, to the aqueous solutions (or aqueous liquids) of drug-encapsulating polymer micelles before said sterilizing filtration. Although not in limitative sense, maltose, trehalose, xylytol, glucose, sucrose, fructose, lactose, mannitol, dextrin and the like can be named as useful saccharides; and as useful polyethylene glycols, those having molecular weights ranging about 1000 - about 35,000, for example, MACROGOL, 1,000, 1,540, 4,000, 6,000, 20,000 and 35,000, can be named. These adjuvants may be contained in the water to be combined with said residue, or may be added after the drug-encapsulating polymer micelles supplied by the residue are dispersed and dissolved in water, and then the whole thing can be given a sterilizing filtration. Thus, according to the invention an adjuvant capable of stabilizing the drug-encapsulating polymer micelles in injections can be added to injections with ease and safety.

Such injections can be not only prepared easily and safely, but also when they are lyophilized, the lyophilized preparations can be re-dissolved or reconstituted with water or aqueous liquid to provide solutions containing the drug-encapsulating polymer micelles, in which aggregation of the micelle particles seldom takes place. So far as we are aware, such lyophilized preparations have not appeared in any literature heretofore, and are novel. Therefore, the present invention also provides the lyophilized preparations.

For the lyophilized preparation to exhibit said function and effect, a saccharide present in the solution prior to the lyophilization is added in such an amount as to make its eventual concentration 0.1 - 15% by weight, while a polyethylene glycol is added to make its eventual concentration 0.5 - 10% by weight. Normally the ratio between the block copolymer and the saccharide or polyethylene glycol lies within a range of 1:1 - 1:10 or 1.05 - 1:10 by weight, respectively.

### Brief Explanation of Drawings

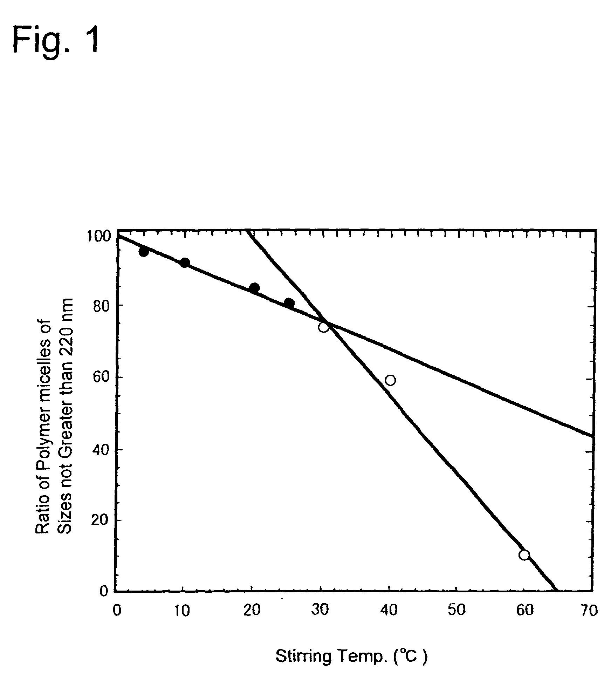

Fig. 1 is a graph showing the changes in the ratio of the particles of sizes not more than 220 nm, in the polymer micelle particles produced in Examples 1-5 and Controls 1 and 2.

### Best Mode for Carrying Out the Invention

Hereinafter the present invention is explained still more specifically, referring to examples in which paclitaxel was used as the sparingly water-soluble drug, it being understood that similar results can be obtained using other drugs.

### Examples 1-5 and Controls 1 and 2

### Block copolymer used:

Polyethylene glycol (molecular weight, 12,000) 50% hydrolyzed benzyl polyaspartate (n=50) (hereinafter referred to as "PEG-PBLA 12-50 P.H. 50%"): 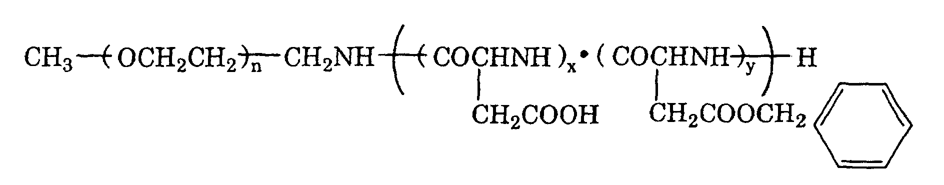 n: a number that makes the molecular weight of the poly(ethylene glycol) about 12,000
x+y: about 50
x/(x + y) = 0.5

Each 20 mg of paclitaxel and each 100 mg of PEG-PBLA 12-50 P.H. 50% were measured out in each one screw bottle, to which 2.0 ml of acetone was added to dissolve the content therein under stirring. Then nitrogen gas was blown thereto to remove most of the acetone, followed by drying under reduced pressure to completely remove the acetone. To each bottle then 10 ml of water was added and sealed airtight. The bottles were violently stirred for one day and one night, at 4°C (Example 1), 10°C (Example 2), 20°C (Example 3), 25°C (Example 4), 30°C (Example 5), 40°C (Control 1) and 60°C (Control 2), respectively, and subjected to an ultrasonic treatment (130 W, 1 sec. pulse, 10 minutes). Samples were taken from the bottles and the particle sizes in the solutions were measured with dynamic light scattering photometer (Otsuka Electronics Co., DLS-7000 DH Model). Further, MACROGOL 4000 was added each to a concentration of 20 mg/ml and maltose, to a concentration of 40 mg/ml and dissolved. The solutions were frozen with dry ice-acetone freezing mixture to provide lyophilized preparations.

Average particle sizes after the ultrasonic treatment were as shown in the following Table 1.

**TABLE 1**

| Example | Stirring temp. (°C) | Average particle size (nm) | Ratio of particles of sizes not more than 220 nm (%)* |
|---|---|---|---|
| Example 1 | 4°C | 92.5 | 94.7 (n=1) |
| Example 2 | 10°C | 119.8 | 91.4 (n=2) |
| Example 3 | 20°C | 139.4 | 84.8 (n=2) |
| Example 4 | 25°C | 146.0 | 80.5 (n=2) |
| Example 5 | 30°C | 138.5 | 73.5 (n=2) |
| Control 1 | 40°C | 165.0 | 58.9 (n=1) |
| Control 2 | 60°C | 360.4 | 10.6 (n=1) |

| | | | |
|---|---|---|---|
| * Result of DLS measurement calculated from G(ls), n: number of test run(s) | | | |

The results as given in Table 1 were plotted with the axis of abscissae representing the stirring temperature and that of ordinates, the ratio of the drug-encapsulating polymer micelles of average particle sizes not more than 220 nm. The result was shown in Fig. 1. The respective regression lines were: (y = 98.888 - 0.78135x R = 0.98474 y = 140.49 - 2.1421x R = 0.99397).

### Industrial Applicability

According to the present invention, medical preparations containing sparingly water-soluble drug-encapsulating polymer micelles of controlled particle size can be efficiently prepared, and the invention is utilizable in the trade of pharmaceutical manufacturers.

## Claims

1. A method for making a preparation containing drug-encapsulating polymer micelles with a controlled size, which comprises forming a solution by dispersing and dissolving a block copolymer with hydrophilic and hydrophobic segments, and a sparingly water-soluble drug in a volatile organic solvent, removing the organic solvent, joining the resultant residue to water, and stirring the same at a temperature not higher than 30°C for a time sufficient to uniformly disperse said residue in the water.

2. The production method according to Claim 1, in which the stirring is conducted at a temperature not higher than 25°C.

3. The production method according to Claim 1, in which the stirring is conducted at a temperature not higher than 10°C.

4. The production method according to Claim 1, which further comprises additional steps of adding adjuvant selected from a group consisting of saccharides and polyethylene glycol to the aqueous mixture before, halfway or after it is stirred for a time sufficient to uniformly disperse said residue in the water; stirring; and subjecting the system to sterilizing filtration.

5. The production method according to Claim 1, in which the stirring is conducted at a temperature not higher than 10°C, and which further comprises the additional steps of adding adjuvant selected from a group consisting of saccharides and polyethylene glycol to the aqueous mixture before, halfway or after it is stirred for a time sufficient to uniformly disperse said residue in the water; stirring, and subjecting the system to sterilizing filtration.

6. The production method according to Claim 5, which further comprises an additional step of lyophilizing the filtrate.

7. The production method according to Claim 1, in which the block copolymer comprises a hydrophilic segment formed of poly(ethylene glycol) and a hydrophobic segment selected from a group consisting of poly(β-alkylaspartate), poly(β-alkylaspartate-co-aspartic acid), poly(β-aralkylaspartate), poly(β-aralkylaspartate-co-aspartic acid), poly(γ-alkylglutamate), poly(γ-alkylglutamate-co-glutamic acid), poly(γ-aralkylglutamate), poly(β-alkylaspartamide), poly(β-alkylaspartamide-co-aspartic acid), poly(β-aralkyl-aspartamide), poly(β-aralkylaspartamide-co-aspartic acid), poly(γ-alkylglutamide), poly(γ-alkylglutamide-co-glutamic acid), poly(γ-aralkylglutamide), poly(γ-aralkylglutamide-co-glutamic acid), poly(lactide), poly(lactide-co-glycolide), poly(ε-caprolactone), poly(δ-valerolactone) and poly(γ-butyrolactone); and the block copolymer is capable of forming polymer micelles in an aqueous medium.

8. The production method according to Claim 1, in which the stirring is conducted at a temperature not higher than 10°C; which method further comprises the additional steps of adding adjuvant selected from a group consisting of saccharides and polyethylene glycol to the aqueous mixture before, halfway or after it is stirred for a time sufficient to uniformly disperse said residue in the water; stirring; and subjecting the system to sterilizing filtration; said copolymer comprising a hydrophilic segment formed of poly(ethylene glycol) and a hydrophobic segment selected from a group consisting of poly(β-alkylaspartate), poly(β-alkylaspartate-co-aspartic acid), poly(β-aralkylaspartate), poly(β-aralkylaspartate-co-aspartic acid), poly(γ-alkylglutamate), poly(γ-alkylglutamate-co-glutamic acid), poly(γ-aralkylglutamate), poly(β-alkylaspartamide), poly(β-alkylaspartamide-co-aspartic acid), poly(β-aralkylaspartamide), poly(β-aralkylaspartamide-co-aspartic acid), poly-(γ-alkylglutamide), poly(γ-alkylglutamide-co-glutamic acid), poly(γ-aralkylglutamide), poly(γ-aralkylglutamide-co-glutamic acid), poly(lactide), poly(lactide-co-glycolide), poly(ε-caprolactone), poly(δ-valerolactone) and poly(γ-butyrolactone); and the block copolymer being capable of forming polymer micelles in an aqueous medium.

9. The production method according to Claim 8, in which the block copolymer is expressed by the following formula (I) or (II): 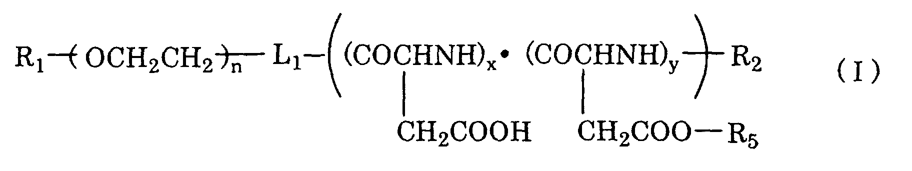 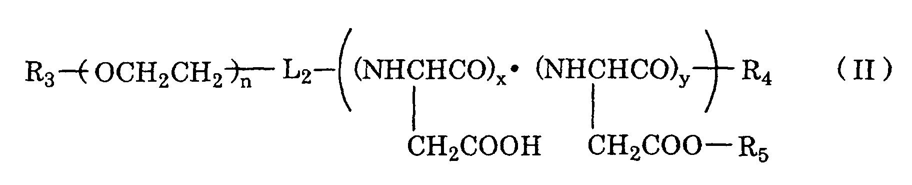 [in the above formulae,
R₁ and R₃ each independently stands for hydrogen or an optionally protected functional group-substituted, or unsubstituted, lower alkyl,
R₂ stands for hydrogen, saturated or unsaturated C₁-C₂₉ aliphatic carbonyl or arylcarbonyl,
R₄ stands for hydroxyl, saturated or unsaturated C₁-C₃₀ aliphatic oxy or aryl-lower alkyloxy,
R₅ stands for benzyl, alkylbenzyl, or allyl,
L₁ and L₂ each independently stands for a linker,
n is an integer of 10-2500, and
x and y are same or different integers, their sum being 10-300, x:y being within a range of 8:2-0:1, and x and y being present each at random].

10. The production method according to Claim 9, in which L₁ is selected from a group consisting of -NH-, -O-, -CO-, -CH₂-, -O-Z-S-Z-, -O-Z-NH- and -OCO-Z-NH- (wherein Z stands for C₁-C₄ alkylene, independently of each other), and
L₂ is selected from a group consisting of -OCO-Z-CO-, -NHCO-Z-CO- and -O-Z-NH- (wherein Z stands for C₁-C₄ alkylene).

11. The production method according to Claim 1, in which the sparingly water-soluble drug is selected from a group consisting of paclitaxel, camptothecin, cisplatin, daunorubicin, methotrexate, mitomycin C, docetaxel, vincristine, amphotericin B, nystatin, prostaglandins and macrolide antibiotics.

12. The production method according to Claim 5, in which the sparingly water-soluble drug is selected from a group consisting of paclitaxel, camptothecin, cisplatin, daunorubicin, methotrexate, mitomycin C, docetaxel, vincristine, amphotericin B, nystatin, prostaglandins and macrolide antibiotics.

13. The production method according to Claim 8, in which the sparingly water-soluble drug is selected from a group consisting of paclitaxel, camptothecin, cisplatin, daunorubicin, methotrexate, mitomycin C, docetaxel, vincristine, amphotericin B, nystatin, prostaglandins and macrolide antibiotics.

14. The production method according to Claim 13, in which said drug and block copolymer are used at a weight ratio within a range of 1:10 - 1:1.

15. The production method according to Claim 1 in which the organic solvent is selected from a group consisting of methyl alcohol, ethyl alcohol, isopropyl alcohol, acetone, acetonitrile, methyl acetate, ethyl acetate, tetrahydrofuran, cyclohexane, diethyl ether, and mixtures of at least two of these.

16. The production method according to Claim 8 in which the organic solvent is selected from a group consisting of methyl alcohl, ethyl alcohol, isopropyl alcohol, acetone, acetonitrile, methyl acetate, ethyl acetate, tetrahydrofuran, cyclohexane, diethyl ether, and mixtures of at least two of these.

17. A lyophilized preparation which contains drug-encapsulating polymer micelles and adjuvant selected from a group consisting of saccharides and poly(ethylene glycol), and which is obtained by a method comprising forming a solution by dispersing and dissolving
a) a block copolymer expressed by the following formula (I) or (II): 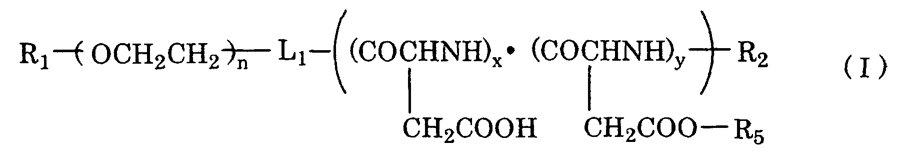 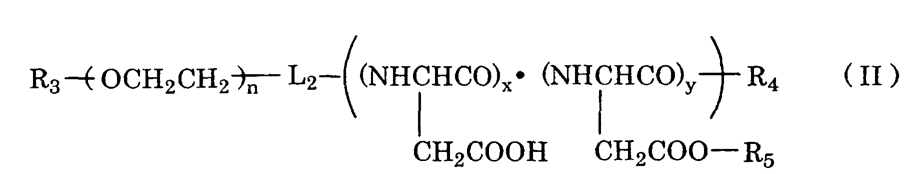 [in the above formulae,
R₁ and R₃ each independently stands for hydrogen or an optionally protected functional group-substituted, or unsubstituted, lower alkyl,
R₂ stands for hydrogen, saturated or unsaturated C₁-C₂₉ aliphatic carbonyl or arylcarbonyl,
R₄ stands for hydroxyl, saturated or unsaturated C₁-C₃₀ aliphatic oxy or aryl-lower alkyloxy,
R₅ stands for benzyl, alkylbenzyl or allyl,
L₁ and L₂ each independently stands for a linker,
n is an integer of 10-2500, and
x and y are same or different integers, their sum being 10-300, x:y being within a range of 8:2-0:1, and x and y being present each at random], and
b) a sparingly water-soluble drug which is selected from a group consisting of paclitaxel, camptothecin, cisplatin, daunorubicin, methotrexate, mitomycin C, docetaxel, vincristine, amphotericin B, nystatin, prostaglandins and macrolide antibiotics,
in a volatile organic solvent; removing the organic solvent; joining the resultant residue to water, and stirring the same at a temperature not higher than 30°C for a time sufficient to uniformly disperse said residue in the water; said method further comprising the steps of adding adjuvant selected from a group consisting of saccharides and polyethylene glycol to the aqueous mixture before, halfway or after it is stirred for a time sufficient to uniformly disperse said residue in the water; stirring; subjecting the system to sterilizing filtration; and lyophilizing the filtrate.

18. The preparation of Claim 17, in which said saccharide is selected from a group consisting of maltose, trehalose, xylytol, glucose, sucrose, fructose, lactose, mannitol and dextrin; and said polyethylene glycol is selected from a group consisting of polyethylene glycols having molecular weight ranging about 1,000 - about 35,000.

19. The preparation of Claim 17, in which said sparingly water-soluble drug is selected from a group consisting of paclitaxel, camptothecin, irinotecan and docetaxel.

20. The preparation of Claim 17, of which all of the drug-encapsulating polymer micelles can substantially pass through a filter of 0.22 µm in pore size, when the lyophilized preparation is reconstituted in an aqueous liquid.
